# EUROPEAN PATENT APPLICATION

(11) **EP 4 799 716 A1**
(43) Date of publication of application: **02.09.2026**
(21) Application number: 26156266.4
(22) Date of filing: 04.02.2026
(51) Int. Cl.: B01D 61/36

(54) **ORGANIC SOLVENT REGENERATOR, SUBSTRATE PROCESSING SYSTEM, AND METHOD OF REGENERATING ORGANIC SOLVENT**

(30) Priority: 26.02.2025 JP 2025028879
(71) Applicant: SCREEN Holdings Co., Ltd., Kyoto-shi, Kyoto 602-8585 (JP)
(72) Inventor: IWAO, Michinori, Kyoto, 602-8585 (JP); YOSHIDA, Yukifumi, Kyoto, 602-8585 (JP); UEMURA, Tomohiro, Kyoto, 602-8585 (JP); MINAMI, Shoyo, Kyoto, 602-8585 (JP); UEDA, Yusuke, Kyoto, 602-8585 (JP); YAMAMOTO, Masaya, Kyoto, 602-8585 (JP)
(74) Representative: Kilian Kilian & Partner mbB

(57) **Abstract**

An organic solvent regenerator includes a collection tank, an exhaust pipe, an exhaust valve, a circulator, a separation pipe, and a controller. An organic solvent and water flow into the collection tank through a collection pipe. The exhaust pipe in which the exhaust valve is disposed is connected to the collection tank. The controller opens the exhaust valve during at least a part of an inflow period in which at least one of the water and the organic solvent flows into the collection tank through the collection pipe, and closes the exhaust valve during at least a part of a separation operation period in which a separation operation of causing the circulator to circulate the mixed liquid and separating the water from the mixed liquid is performed.

## Description

### BACKGROUND

### Technical Field

The present disclosure relates to an organic solvent regenerator, a substrate processing system, and a method of regenerating an organic solvent.

### Description of the Background Art

Japanese Patent Application Laid-Open No. 2017-41505 discloses an IPA collection system. The IPA collection system collects aqueous isopropyl alcohol (IPA) discharged from processing units that process substrates. The IPA collection system includes a storage tank, a circulation pipe, a pump, and a dewatering unit. The aqueous IPA from the processing units is supplied to the storage tank. The circulation pipe is connected to the storage tank, and returns the aqueous IPA from the storage tank to the storage tank. The pump is disposed in the circulation pipe, and sends the aqueous IPA from an upstream end toward a downstream end of the circulation pipe. The dewatering unit is disposed in the circulation pipe. The dewatering unit includes a concentration chamber, a permeation chamber, and a separation membrane that partitions the concentration chamber and the permeation chamber. The concentration chamber is inserted into the circulation pipe. The separation membrane allows water from the concentration chamber to pass through the permeation chamber, and does not allow the IPA to pass through the permeation chamber.

The collection system circulates the aqueous IPA through a circulation path including the storage tank and the circulation pipe. This circulation allows the aqueous IPA to pass through the concentration chamber of the dewatering unit. An inflow of the water in the aqueous IPA into the permeation chamber through the separation membrane increases an IPA concentration of the aqueous IPA during the circulation. In other words, this circulation allows the aqueous IPA having a high IPA concentration to be stored in the storage tank. The aqueous IPA in this storage tank is again supplied to the processing units. This can reduce a waste amount of the IPA.

In Japanese Patent Application Laid-Open No. 2017-41505, a supply pipe and an exhaust pipe are connected to the storage tank. Gas is supplied to the storage tank through the supply pipe, and the gas in the storage tank is discharged to an exterior through the exhaust pipe. A relief valve disposed in the exhaust pipe maintains constant the pressure in the storage tank.

### SUMMARY

In Japanese Patent Application Laid-Open No. 2017-41505, IPA vapors are generated from the aqueous IPA in the storage tank. Since gas in the storage tank is discharged to an exterior through the exhaust pipe, these IPA vapors are also discharged to the exterior through the exhaust pipe. This creates a problem of an increase in a waste amount of the IPA.

When the exhaust pipe is always closed, a problem arises in which the aqueous IPA from the processing units hardly flows into the storage tank.

Thus, the present disclosure has an object of providing a technology that allows a mixed liquid of an organic solvent and water from a substrate processing apparatus to easily flow into a collection tank, and that can reduce a waste amount of the organic solvent.

According to one aspect, an organic solvent regenerator includes: a collection tank into which an organic solvent and water flow through a collection pipe and which stores a mixed liquid of the organic solvent and the water, the organic solvent and the water being discharged from a substrate processing apparatus that processes a substrate; an exhaust pipe connected to the collection tank; an exhaust valve disposed in the exhaust pipe; a circulator including a circulation pipe connected to the collection tank, and a membrane separator disposed in the circulation pipe, the membrane separator separating the water from the mixed liquid; a separation pipe which is connected to the membrane separator and through which the water separated by the membrane separator flows; and a controller that opens the exhaust valve during at least a part of an inflow period in which at least one of the water and the organic solvent flows into the collection tank through the collection pipe and that closes the exhaust valve during at least a part of a separation operation period in which a separation operation of causing the circulator to circulate the mixed liquid and separating the water from the mixed liquid is performed.

According to one aspect, a substrate processing system includes the organic solvent regenerator and the substrate processing apparatus.

According to one aspect, a method of regenerating an organic solvent includes: opening an exhaust valve disposed in an exhaust pipe connected to a collection tank during at least a part of an inflow period in which at least one of an organic solvent and water flows into the collection tank through a collection pipe, the organic solvent and the water being discharged from a substrate processing apparatus that processes a substrate; and closing the exhaust valve during at least a part of a separation operation period in which a separation operation of causing a circulator to circulate a mixed liquid of the organic solvent and the water and separating the water from the mixed liquid is performed, the circulator including a circulation pipe connected to the collection tank storing the mixed liquid, and a membrane separator disposed in the circulation pipe and separating the water from the mixed liquid.

These and other objects, features, aspects, and advantages of the present disclosure will become more apparent from the following detailed description of the present disclosure when taken in conjunction with the accompanying drawings.

### BRIEF DESCRIPTION OF THE DRAWINGS

FIG. 1 is a diagram schematically illustrating one example structure of a substrate processing system including an organic solvent regenerator according to Embodiment 1;
FIG. 2 is a block diagram schematically illustrating one example configuration of a controller;
FIG. 3 is a timing chart illustrating an example of closing and opening of a collection valve and an exhaust valve;
FIG. 4 is a diagram schematically illustrating one example state of the organic solvent regenerator during an inflow period;
FIG. 5 is a diagram schematically illustrating one example state of the organic solvent regenerator during a separation operation;
FIG. 6 is a diagram schematically illustrating one example structure of an organic solvent regenerator according to Embodiment 2;
FIG. 7 is a flowchart illustrating example pressure control during the separation operation;
FIG. 8 is a diagram schematically illustrating one example structure of an organic solvent regenerator according to Embodiment 3;
FIG. 9 is a diagram schematically illustrating an example structure of a substrate processing apparatus;
FIG. 10 is a flowchart illustrating example operations of an organic solvent regenerator according to Embodiment 4; and
FIG. 11 is a timing chart illustrating example operations of a substrate processing system according to Embodiment 4.

### DESCRIPTION OF THE EMBODIMENTS

Embodiments will be described in detail below with reference to drawings. It should be noted that dimensions and the number of parts are shown in exaggeration or in simplified form as appropriate for the sake of easier understanding. The same reference signs are assigned to parts having similar structures and functions, and overlapping description will be omitted in the following description.

Even when the ordinal numbers such as "first" and "second" are used in the following description, these terms are used for convenience to facilitate the understanding of the details of Embodiments. The order that can be indicated by these ordinal numbers does not restrict the details of Embodiments.

Unless otherwise noted, the expressions indicating relative or absolute positional relationships (e.g., "in one direction", "along one direction", "parallel", "orthogonal", "central", "concentric", and "coaxial") include those exactly indicating the positional relationships and those where an angle or a distance is relatively changed within tolerance or to the extent that similar functions can be obtained. Unless otherwise noted, the expressions indicating equality (e.g., "same", "equal", and "homogeneous") include those indicating quantitatively exact equality and those in the presence of a difference within tolerance or to the extent that similar functions can be obtained. Unless otherwise noted, the expressions indicating shapes (e.g., "rectangular" or "cylindrical") include those indicating geometrically exact shapes and those indicating, for example, roughness or a chamfer to the extent that similar advantages can be obtained. An expression "comprising", "including", "containing", or "having" one constituent element is not an exclusive expression for excluding the presence of the other constituent elements. An expression "at least one of A, B, and C" involves "only A", "only B", "only C", "any two of A, B, and C", and "all of A, B, and C".

### [Embodiment 1]

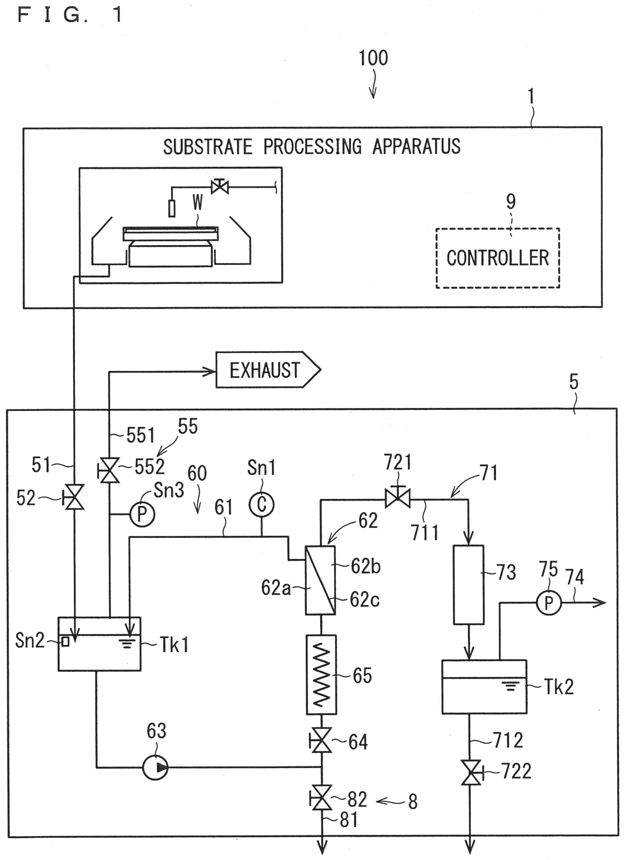

FIG. 1 is a diagram schematically illustrating one example structure of a substrate processing system 100 including an organic solvent regenerator 5 according to Embodiment 1.

The substrate processing system 100 includes a substrate processing apparatus 1, the organic solvent regenerator 5, and a controller 9. The substrate processing apparatus 1 is a processing apparatus that performs a wet process on a substrate W. The substrate processing apparatus 1 may be a single-wafer processing apparatus that processes substrates W one by one, or a batch processing apparatus that processes a plurality of substrates W in one batch. In the example of FIG. 1, the single-wafer processing apparatus is schematically illustrated.

Examples of the substrates W include a semiconductor wafer, a liquid crystal display substrate, an organic electroluminescence (EL) substrate, a flat panel display (FPD) substrate, an optical display substrate, a magnetic disk substrate, an optical disk substrate, a magneto-optical disk substrate, a photomask substrate, and a solar cell substrate. The substrates W have a thin plate shape. In the following, the substrates W are semiconductor wafers. For example, the substrates W are silicon substrates. The substrates W are, for example, disk-shaped. Each of the substrates W has a diameter of, for example, approximately 300 mm, and has a thickness of, for example, approximately more than or equal to 0.5 mm and less than or equal to 3 mm.

The substrate processing apparatus 1 supplies various processing liquids to the substrates W, and performs, on the substrates W, processes corresponding to the types of the processing liquids. While Embodiment 4 will outline an example specific structure of the substrate processing apparatus 1, for example, the substrate processing apparatus 1 supplies the substrate W with pure water (deionized water) and an organic solvent. For example, the substrate processing apparatus 1 supplies the substrate W with pure water, and then supplies the substrate W with an organic solvent. The substrate processing apparatus 1 supplies the substrate W with pure water, so that an object (e.g., a solid such as particles or a liquid such as a chemical solution) adhering to the substrate W can be rinsed out with the pure water. Next, the substrate processing apparatus 1 supplies the substrate W with an organic solvent, so that the liquid adhering to the substrate W can be replaced from the pure water with a rinse liquid. The organic solvent is, for example, an organic solvent more volatile than pure water or an organic solvent with a low surface tension, and is isopropyl alcohol (IPA) as an specific example. Next, the substrate processing apparatus 1 dries the substrate W. When the substrate W is dried, the organic solvent with high volatility and a low surface tension adheres to the substrate W. Thus, the substrate processing apparatus 1 can dry the substrate W more promptly or while avoiding pattern collapse of the substrate W.

A mixed liquid of an organic solvent and water which have been used for processing the substrate W is discharged from the substrate processing apparatus 1 to the organic solvent regenerator 5 through a collection pipe 51. In other words, the collection pipe 51 connects the substrate processing apparatus 1 to the organic solvent regenerator 5. The substrate processing apparatus 1 is, for example, provided upstairs (above the floor) in a factory, and the organic solvent regenerator 5 is, for example, provided downstairs (under the floor) in the factory.

The organic solvent regenerator 5 performs a separation operation of separating water from the mixed liquid to increase a concentration of the organic solvent in the mixed liquid. An example detailed structure and example detailed operations of the organic solvent regenerator 5 will be described later in detail. Hereinafter, the concentration of the organic solvent in the mixed liquid may be referred to as a solvent concentration.

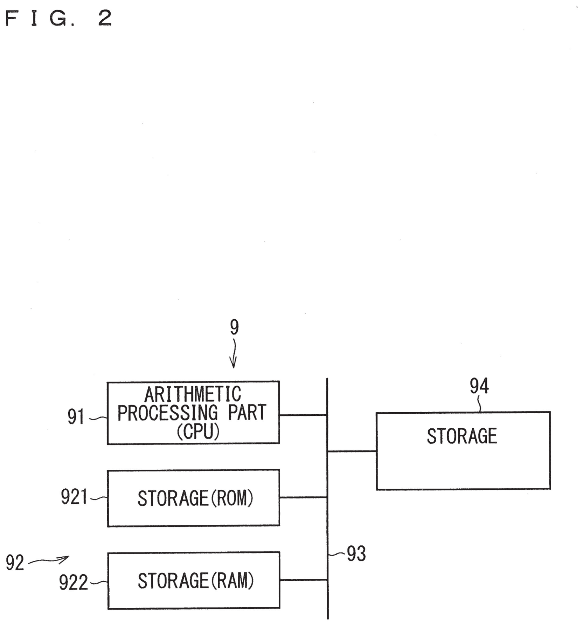

The controller 9 controls the substrate processing system 100. In other words, the controller 9 controls various structures of the substrate processing apparatus 1 and the organic solvent regenerator 5. FIG. 2 is a block diagram schematically illustrating one example configuration of the controller 9. The controller 9 is an electronic circuit, and includes, for example, an arithmetic processing part 91 and a storage 92. In a specific example of FIG. 2, the arithmetic processing part 91 and the storage 92 are mutually connected through a bus 93. The arithmetic processing part 91 is, for example, a central processing unit (CPU). The storage 92 may include a non-transitory storage (e.g., a read-only memory (ROM)) 921 and a transitory storage (e.g., a random-access memory (RAM)) 922. The non-transitory storage 921 may store, for example, a program for defining processes to be executed by the controller 9. The arithmetic processing part 91 executes this program, so that the controller 9 can execute the processes defined in the program. Obviously, hardware such as a dedicated logic circuit may execute a part or all the processes to be executed by the controller 9.

As illustrated in FIG. 2, the controller 9 may be electrically connected to a storage 94. The storage 94 is a non-transitory storage, and may be, for example, a memory or a hard disk.

As illustrated in FIG. 1, the organic solvent regenerator 5 includes a collection tank Tk1, a circulator 60, a separation pipe 71, and a gas discharge part 55.

The collection tank Tk1 is connected to a downstream end of the collection pipe 51. The mixed liquid discharged from the substrate processing apparatus 1 flows into the collection tank Tk1 through the collection pipe 51. The collection tank Tk1 stores the mixed liquid. The collection tank Tk1 is a closed tank. In the example of FIG. 1, a collection valve 52 is disposed in the collection pipe 51. The collection valve 52 is controlled by the controller 9, and switches between closing and opening of the collection pipe 51.

Since the mixed liquid of the organic solvent and water which have been used for processing the substrate W flows into the collection tank Tk1, a solvent concentration of the mixed liquid in the collection tank Tk1 is initially low. Hereinafter, the solvent concentration of the mixed liquid before a separation operation is executed may be referred to as an initial concentration. For example, the initial concentration may be 80% or lower, 70% or lower, or 60% or lower. The initial concentration may be much lower than these.

The gas discharge part 55 discharges the gas in the collection tank Tk1 to an exterior exhaust facility (e.g., an exhaust facility of a factory utility). The gas discharge part 55 includes a first exhaust pipe 551 (corresponding to an exhaust pipe), and an exhaust valve 552. An upstream end of the first exhaust pipe 551 is connected to the collection tank Tk1. In the example of FIG. 1, the upstream end of the first exhaust pipe 551 is connected to a ceiling of the collection tank Tk1. An upstream port of the first exhaust pipe 551 is opened above the liquid level of the mixed liquid in the collection tank Tk1. The exhaust valve 552 is disposed in the first exhaust pipe 551. The exhaust valve 552 is controlled by the controller 9, and switches between closing and opening of the first exhaust pipe 551.

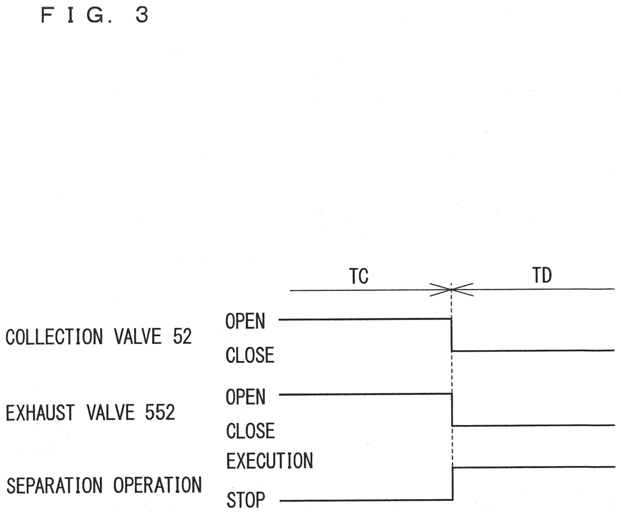

The controller 9 opens the exhaust valve 552 during at least a part of an inflow period in which at least one of the pure water and the organic solvent from the substrate processing apparatus 1 flows into the collection tank Tk1 through the collection pipe 51. FIG. 3 is a timing chart illustrating an example of closing and opening of the collection valve 52 and the exhaust valve 552. During a collectable period TC in which the collection valve 52 is open, the mixed liquid discharged from the substrate processing apparatus 1 can flow into the collection tank Tk1. In other words, during a period in which the substrate processing apparatus 1 discharges the mixed liquid to the collection pipe 51 in the collectable period TC, the mixed liquid flows into the collection tank Tk1 through the collection pipe 51. This period is the inflow period. In the example of FIG. 3, the controller 9 opens the exhaust valve 552 during the entirety of the collectable period TC. Thus, the exhaust valve 552 is open during the entirety of the collectable period TC in the example of FIG. 3. FIG. 3 also illustrates a separation operation, which will be described later in detail.

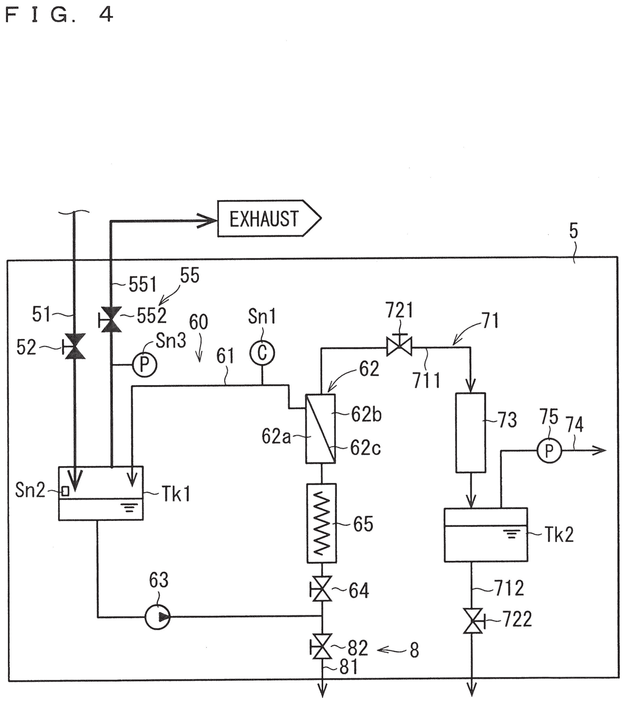

FIG. 4 is a diagram schematically illustrating one example state of the organic solvent regenerator 5 during the inflow period. FIG. 4 illustrates pipes through which a fluid flows using thick lines, and illustrates open valves using solid valves. In the example of FIG. 4, the collection valve 52 is open. Once the substrate processing apparatus 1 discharges the mixed liquid to the collection pipe 51, the mixed liquid can flow into the collection tank Tk1 through the collection pipe 51. Upon an inflow of the mixed liquid into the collection tank Tk1, the storage amount of the mixed liquid in the collection tank Tk1 increases with time. Conversely speaking, the volume of gas space occupied by gas in the collection tank Tk1 decreases with time. Thus, the exhaust valve 552 is also open in the example of FIG. 4. Therefore, the gas in the collection tank Tk1 is discharged through the first exhaust pipe 551. Consequently, a pressure rise in the collection tank Tk1 upon an inflow of the mixed liquid into the collection tank Tk1 can be mitigated or avoided. Thus, the mixed liquid can easily flow into the collection tank Tk1.

The organic solvent regenerator 5 may include a storage amount sensor Sn2 with reference to FIG. 1. The storage amount sensor Sn2 measures a storage amount of the mixed liquid in the collection tank Tk1, and outputs an electrical signal indicating a result of the measurement to the controller 9. The storage amount sensor Sn2 may be a liquid level sensor that detects a height position of a liquid level of the mixed liquid in the collection tank Tk1. The controller 9 may perform a separation operation when the storage amount of the mixed liquid in the collection tank Tk1 is larger than or equal to a predetermined reference collection amount.

The circulator 60 includes the circulation pipe 61 and a membrane separator 62. The circulation pipe 61 is connected to the collection tank Tk1. The circulation pipe 61 is a pipe that returns the mixed liquid from the collection tank Tk1 to the collection tank Tk1. In other words, an upstream end and a downstream end of the circulation pipe 61 are connected to the collection tank Tk1. The collection tank Tk1 and the circulation pipe 61 form a circulation path through which the mixed liquid circulates. In the example of FIG. 1, the upstream end of the circulation pipe 61 is connected to a bottom of the collection tank Tk1, and the downstream end of the circulation pipe 61 is connected to the ceiling of the collection tank Tk1.

In the example of FIG. 1, a liquid feeder 63 is disposed in the circulation pipe 61. The liquid feeder 63 allows a processing liquid to flow from the upstream end toward the downstream end of the circulation pipe 61. The liquid feeder 63 is, for example, a liquid pump, and is controlled by the controller 9. The liquid feeder 63 may be, for example, a magnetic levitation pump.

In the example of FIG. 1, a circulation valve 64 is disposed in the circulation pipe 61. The circulation valve 64 is controlled by the controller 9, and switches between closing and opening of the circulation pipe 61. In the example of FIG. 1, the circulation valve 64 is disposed on the downstream end side of the circulation pipe 61 with respect to the liquid feeder 63.

The membrane separator 62 is disposed in the circulation pipe 61, and separates water from the mixed liquid. Specifically, the membrane separator 62 includes a casing, and includes a first path 62a, a second path 62b, and a separation membrane 62c inside the casing. The first path 62a is inserted into the circulation pipe 61, and constitutes a part of the circulation path of the circulator 60. For this reason, the mixed liquid passes through the first path 62a. The separation membrane 62c partitions the first path 62a and the second path 62b. The separation membrane 62c is a membrane that allows water in the mixed liquid to pass from the first path 62a to the second path 62b, and substantially blocks the organic solvent. Consequently, the membrane separator 62 separates water from the mixed liquid.

The separation membrane 62c may be a zeolite membrane, an organic separation membrane, or a carbon nanotube (CNT) separation membrane. For example, the zeolite membrane has a crystal structure in which (SiO4)⁴⁻ and (AlO4)⁵⁻ having a tetrahedral structure are mutually coupled. The organic separation membrane is, for example, an organic film made of polyvinyl alcohol, chitosan, or polyimide. The CNT separation membrane is, for example, a membrane obtained by adding carbon nanotubes to a membrane made of polyamide or the like. Alternatively, a two-dimensional material may be adopted as a material of the separation membrane 62c. The two-dimensional material is a material composed of one layer of atoms, and, for example, may be molybdenum sulfide (MoS₂) or a composite atomic layer compound including an early transition metal (titanium, vanadium, or the like) and a light element (carbon or nitrogen). Alternatively, a metal organic framework (MOF) material or a carbon material (for example, graphene or graphene oxide) may be applied as a material of the separation membrane 62c. Here, a zeolite membrane is applied as the separation membrane 62c.

The separation pipe 71 is connected to the membrane separator 62. Water separated by the membrane separator 62 flows through the separation pipe 71. Specifically, an upstream end of the separation pipe 71 is connected to the second path 62b of the membrane separator 62. Thus, water passing through the separation membrane 62c flows into the separation pipe 71. Hereinafter, a fluid separated from the mixed liquid by the membrane separator 62 may be referred to as a separation fluid. The separation fluid contains water as a main component. The separation fluid can slightly contain an organic solvent.

A decompression pump 75 is provided in the example of FIG. 1. The decompression pump 75 reduces the pressure in the second path 62b through the separation pipe 71. In the example of FIG. 1, a separation tank Tk2 is disposed in the separation pipe 71. The separation fluid separated by the membrane separator 62 flows into the separation tank Tk2. The separation tank Tk2 stores the separation fluid. In the separation pipe 71, a portion between the membrane separator 62 and the separation tank Tk2 may be referred to as a piping portion 711, and a portion downstream of the separation tank Tk2 may be referred to as a piping portion 712 hereinafter. In the example of FIG. 1, an upstream end of the piping portion 711 is connected to the second path 62b, and a downstream end of the piping portion 711 is connected to a ceiling of the separation tank Tk2. A downstream port of the piping portion 711 is opened above a liquid level of the separation fluid in the separation tank Tk2. In the example of FIG. 1, an upstream end of the piping portion 712 is connected to a bottom of the separation tank Tk2.

In the example of FIG. 1, an upstream end of a decompression pipe 74 is also connected to the separation tank Tk2. For example, the upstream end of the decompression pipe 74 is connected to the ceiling of the separation tank Tk2. An upstream port of the decompression pipe 74 is opened above the liquid level of the separation fluid in the separation tank Tk2. The decompression pipe 74 is connected to the decompression pump 75. The decompression pump 75 is controlled by the controller 9. Upon the decompression pump 75 being operated, the decompression pump 75 suctions gas in the second path 62b of the membrane separator 62 through the piping portion 711, the separation tank Tk2, and the decompression pipe 74 in this order. Consequently, the pressure in the second path 62b of the membrane separator 62 decreases, and the water in the mixed liquid in the first path 62a is effectively suctioned by the second path 62b through the separation membrane 62c.

In the example of FIG. 1, a separation valve 721 is disposed in the piping portion 711 of the separation pipe 71. The separation valve 721 is controlled by the controller 9, and switches between closing and opening of the piping portion 711. In the example of FIG. 1, a separation valve 722 is disposed in the piping portion 712 of the separation pipe 71. The separation valve 722 is controlled by the controller 9, and switches between closing and opening of the piping portion 712.

In the example of FIG. 1, a temperature regulator 65 is disposed in the circulation pipe 61. The temperature regulator 65 regulates the temperature of the mixed liquid flowing through the circulation pipe 61. The temperature regulator 65 may include, for example, a heater. The heater may be an electric resistance heater with an electric heating wire, an optical heater that emits light (e.g., infrared rays) for heating, or an electronic cooling/heating unit including a Peltier element. The temperature regulator 65 heats the mixed liquid, so that the mixed liquid at a high temperature can flow into the membrane separator 62. The higher the temperature is, the higher the velocity of molecules in the mixed liquid is. Thus, water molecules in the mixed liquid at a high temperature easily pass through the separation membrane 62c. This allows the membrane separator 62 to separate the separation fluid from the mixed liquid with a higher degree of efficiency. The temperature regulator 65 is controlled by the controller 9. The controller 9 controls the temperature regulator 65 such that the temperature of the mixed liquid falls within a temperature range appropriate for the separation operation.

In the example of FIG. 1, the membrane separator 62 is disposed on the downstream end side of the circulation pipe 61 with respect to the circulation valve 64. Furthermore, in the example of FIG. 1, the temperature regulator 65 is disposed on the upstream end side of the circulation pipe 61 with respect to the membrane separator 62. Since the temperature regulator 65 is disposed on the inflow side of the membrane separator 62, the temperature of the mixed liquid flowing into the membrane separator 62 can be regulated with higher accuracy. As a specific example, the temperature regulator 65 can be disposed between the circulation valve 64 and the membrane separator 62.

When the temperature regulator 65 heats the mixed liquid, much more steam of the mixed liquid can flow through the circulation pipe 61. In other words, much more water vapors and steam of the organic solvent can flow through the circulation pipe 61. Thus, the water vapors can pass through the separation membrane 62c of the membrane separator 62, and may flow into the separation pipe 71. In other words, the separation fluid can contain the water vapors.

Thus, as illustrated in FIG. 1, a cooler 73 may be disposed in the piping portion 711 of the separation pipe 71. The separation fluid flows into the cooler 73. The cooler 73 cools and condenses the separation fluid. The cooler 73 may include, for example, a heat exchanger (not illustrated), and a coolant supply source (not illustrated) that supplies a coolant to the heat exchanger. The heat exchanger includes a separation path through which the separation fluid passes, and a coolant path through which a coolant passes. The separation fluid is cooled by heat exchange between the separation fluid in the separation path and the coolant in the coolant path. The coolant supply source cools the coolant flowing from the heat exchanger, and supplies the cooled coolant to the heat exchanger. The coolant supply source may be, for example, a heat pump. The separation fluid condensed by the cooler 73 (i.e., a separated liquid) is supplied to the separation tank Tk2, and is stored in the separation tank Tk2.

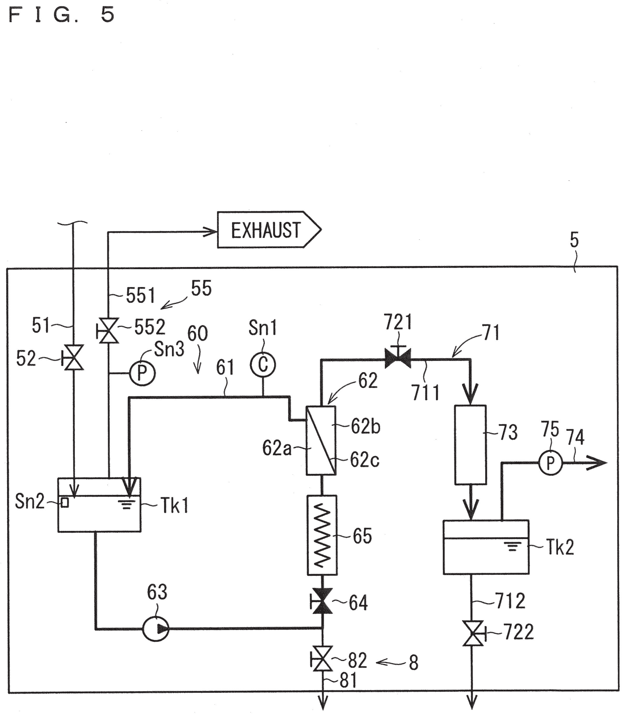

The controller 9 controls various structures of the organic solvent regenerator 5 such that the separation operation is performed. FIG. 5 is a diagram schematically illustrating one example state of the organic solvent regenerator 5 during the separation operation. In the example of FIG. 5, the controller 9 opens the circulation valve 64 and the separation valve 721, and operates the temperature regulator 65, the liquid feeder 63, the cooler 73, and the decompression pump 75 such that the separation operation is performed. This circulation continues to route the mixed liquid through the membrane separator 62. Since the decompression pump 75 reduces the pressure in the second path 62b, mainly water (containing water vapors) in the mixed liquid is suctioned from the first path 62a to the second path 62b through the separation membrane 62c. Consequently, the membrane separator 62 continues to separate the separation fluid (mainly water) from the mixed liquid, and the separation fluid continues to be discharged through the separation pipe 71. Thus, a solvent concentration of the mixed liquid in the circulation path increases with time.

In the example of FIG. 1, the organic solvent regenerator 5 includes a concentration sensor Sn1. The concentration sensor Sn1 measures the solvent concentration of the mixed liquid, and outputs an electrical signal indicating a result of the measurement to the controller 9. In the example of FIG. 1, the concentration sensor Sn1 is disposed in the circulation pipe 61. As a specific example, the concentration sensor Sn1 is disposed on the downstream end side of the circulation pipe 61 with respect to the membrane separator 62. The concentration sensor Sn1 measures the solvent concentration of the mixed liquid flowing through the circulation pipe 61. Since the solvent concentration of the mixed liquid flowing through the circulation pipe 61 is almost equal to the solvent concentration of the mixed liquid in the collection tank Tkl, the concentration sensor Sn1 can indirectly measure the solvent concentration of the mixed liquid in the collection tank Tk1. The controller 9 may stop the separation operation when the solvent concentration measured by the concentration sensor Sn1 is higher than or equal to a target concentration. Consequently, the mixed liquid with the solvent concentration higher than or equal to the target concentration is stored in the collection tank Tk1. Hereinafter, the mixed liquid with the solvent concentration higher than or equal to the target concentration may be referred to as a concentrate.

The controller 9 closes the exhaust valve 552 during at least a part of a separation operation period TD in which the separation operation is performed. This can reduce an amount of steam of the organic solvent to be discharged to an exterior through the first exhaust pipe 551 during the separation operation. Thus, a waste amount of the organic solvent can be reduced. In the example of FIG. 3, the controller 9 closes the exhaust valve 552 during the entirety of the separation operation period TD (see also FIG. 5). Thus, a discharge amount of the steam of the organic solvent during the separation operation can be further reduced.

As illustrated in FIG. 3, the controller 9 may close the collection valve 52 during the separation operation period TD. The controller 9 can close the collection valve 52 during the entirety of the separation operation period TD. This can avoid an inflow of a mixed liquid with a low solvent concentration into the collection tank Tk1 during the separation operation period.

When the collection valve 52 and the exhaust valve 552 are closed during the entirety of the separation operation period TD, the circulation path formed by the collection tank Tk1 and the circulation pipe 61 is sealed during the entirety of the separation operation period TD. Thus, the mixed liquid circulates through the sealed circulation path during the entirety of the separation operation period TD. Thus, a discharge amount of the steam of the organic solvent can be reduced the most.

The substrate processing apparatus 1 can discharge the mixed liquid during the separation operation period TD. Thus, the organic solvent regenerator 5 may include another collection tank (not illustrated). Another collection pipe branching off from the collection pipe 51 may be connected to the other collection tank. Another collection valve may be disposed in the other collection pipe. The controller 9 may open the other collection valve when the collection valve 52 is closed. This allows the mixed liquid discharged from the substrate processing apparatus 1 to be collected in the other collection tank during the separation operation period TD.

In the example of FIG. 1, the organic solvent regenerator 5 includes a concentrate supply part 8. The concentrate supply part 8 supplies the concentrate in the collection tank Tk1 to an exterior. The exterior herein means an exterior of the organic solvent regenerator 5. The concentrate supply part 8 may supply the substrate processing apparatus 1 with the concentrate. The substrate processing apparatus 1 supplies the substrate W with the concentrate. Consequently, the substrate processing system 100 can recycle the organic solvent. In other words, the organic solvent regenerator 5 contributes to saving of the organic solvent.

In the example of FIG. 1, the concentrate supply part 8 includes a supply pipe 81 and a supply valve 82. In the example of FIG. 1, an upstream end of the supply pipe 81 is connected to the circulation pipe 61 between the liquid feeder 63 and the circulation valve 64. A downstream end of the supply pipe 81 is connected to an exterior. The supply valve 82 is disposed in the supply pipe 81. The supply valve 82 is controlled by the controller 9, and switches between closing and opening of the supply pipe 81.

The controller 9 opens the supply valve 82 with the concentrate being stored in the collection tank Tk1, and operates the liquid feeder 63. Consequently, the concentrate in the collection tank Tk1 is supplied to an exterior through the supply pipe 81. Here, the controller 9 can stop the circulation by the circulator 60. In other words, the organic solvent regenerator 5 can supply the concentrate to an exterior while stopping the separation operation. The upstream end of the supply pipe 81 may be connected to, for example, the bottom of the collection tank Tk1. Here, a liquid feeder different from the liquid feeder 63 is provided in the supply pipe 81.

As described above, the controller 9 opens the exhaust valve 552 during at least a part of the inflow period of the collectable period TC (see also FIG. 3). Upon opening the exhaust valve 552, the mixed liquid discharged from the substrate processing apparatus 1 can more easily flow into the collection tank Tk1. A period in which the exhaust valve 552 is opened may be, for example, half the inflow period or longer, 80% of the inflow period or more, 90% of the inflow period or more, or the entirety of the inflow period.

The controller 9 closes the exhaust valve 552 during at least a part of the separation operation period TD (see also FIG. 3). Thus, the organic solvent regenerator 5 can reduce a discharge amount of the steam of the organic solvent through the first exhaust pipe 551 during the separation operation, and reduce a waste amount of the organic solvent. A period in which the exhaust valve 552 is closed may be, for example, half the separation operation period TD or longer, 80% of the separation operation period TD or more, 90% of the separation operation period TD or more, or the entirety of the separation operation period TD. When the exhaust valve 552 is closed during the entirety of the separation operation period TD, the organic solvent regenerator 5 can avoid discharge of the steam of the organic solvent through the first exhaust pipe 551 during the separation operation.

When the temperature regulator 65 heats the mixed liquid as described particularly in the specific examples, much more steam of the organic solvent flows through the circulation pipe 61. When such more steam of the organic solvent is discharged to an exterior, a waste amount of the organic solvent increases. In other words, a reuse rate of the organic solvent decreases. Thus, the organic solvent regenerator 5 including the temperature regulator 65 makes Embodiment 1 particularly useful.

In the aforementioned examples, the collection valve 52 is closed during the separation operation period TD. Thus, the organic solvent regenerator 5 can avoid an inflow of the mixed liquid with a low solvent concentration into the collection tank Tk1 during the separation operation. When the collection valve 52 and the exhaust valve 552 are closed during the entirety of the separation operation period TD, the organic solvent regenerator 5 can avoid discharge of the steam of the organic solvent through the circulation path during the separation operation.

### [Embodiment 2]

The membrane separator 62 continues to separate the separation fluid (mainly water) from the mixed liquid in the separation operation. In other words, the separation fluid branches off from the circulation path and continues to flow into the separation pipe 71. Thus, an amount of the mixed liquid circulating through the circulation path (hereinafter also referred to as "an amount of circulation") decreases with time. Thus, the pressure of gas in the circulation path decreases with time. In other words, the pressure in the collection tank Tk1 decreases with time. Thus, the collection tank Tk1 needs to have breakdown voltage performance enough to withstand low pressures. The collection tank Tk1 is expensive.

Thus, Embodiment 2 is designed to mitigate or avoid a decrease in the pressure in the collection tank Tk1 in a region lower than a first reference pressure value.

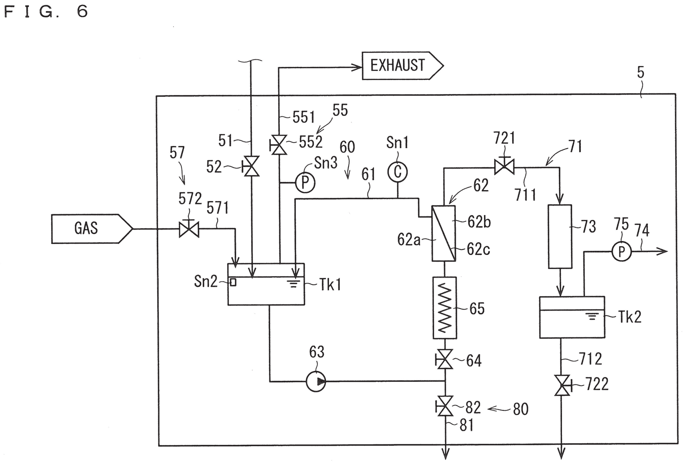

FIG. 6 is a diagram schematically illustrating one example structure of the organic solvent regenerator 5 according to Embodiment 2. The organic solvent regenerator 5 according to Embodiment 2 differs from that according to Embodiment 1 in the presence or absence of a gas supply part 57. The gas supply part 57 supplies gas to the collection tank Tk1. The gas includes, for example, an inert gas. The inert gas includes, for example, at least one of a nitrogen gas and a noble gas. The noble gas includes, for example, an argon gas.

In the example of FIG. 6, the gas supply part 57 includes a first gas supply pipe 571 and a gas supply valve 572. A downstream end of the first gas supply pipe 571 is connected to the collection tank Tk1. In the example of FIG. 6, the downstream end of the first gas supply pipe 571 is connected to the ceiling of the collection tank Tk1. A downstream port of the first gas supply pipe 571 is opened above the liquid level of the mixed liquid in the collection tank Tk1. An upstream end of the first gas supply pipe 571 is connected to a gas supply source. The gas supply source includes a reservoir (a tank) that reserves gas (e.g., an inert gas). The gas supply valve 572 is disposed in the first gas supply pipe 571. The gas supply valve 572 is controlled by the controller 9, and switches between closing and opening of the first gas supply pipe 571. Once the gas supply valve 572 is opened, the gas flows toward the collection tank Tk1 through the first gas supply pipe 571, and flows into the collection tank Tk1. Consequently, the pressure in the collection tank Tk1 can be raised.

In the example of FIG. 6, the organic solvent regenerator 5 includes a pressure sensor Sn3. The pressure sensor Sn3 measures a pressure in the collection tank Tk1. In the example of FIG. 6, the pressure sensor Sn3 is disposed upstream of the exhaust valve 552 in the first exhaust pipe 551. In other words, the pressure sensor Sn3 is disposed between the exhaust valve 552 and the collection tank Tk1. The pressure sensor Sn3 measures a pressure in the first exhaust pipe 551. Since the pressure in a piping portion upstream of the exhaust valve 552 in the first exhaust pipe 551 is almost equal to that in the collection tank Tk1, the pressure sensor Sn3 can indirectly measure the pressure in the collection tank Tk1.

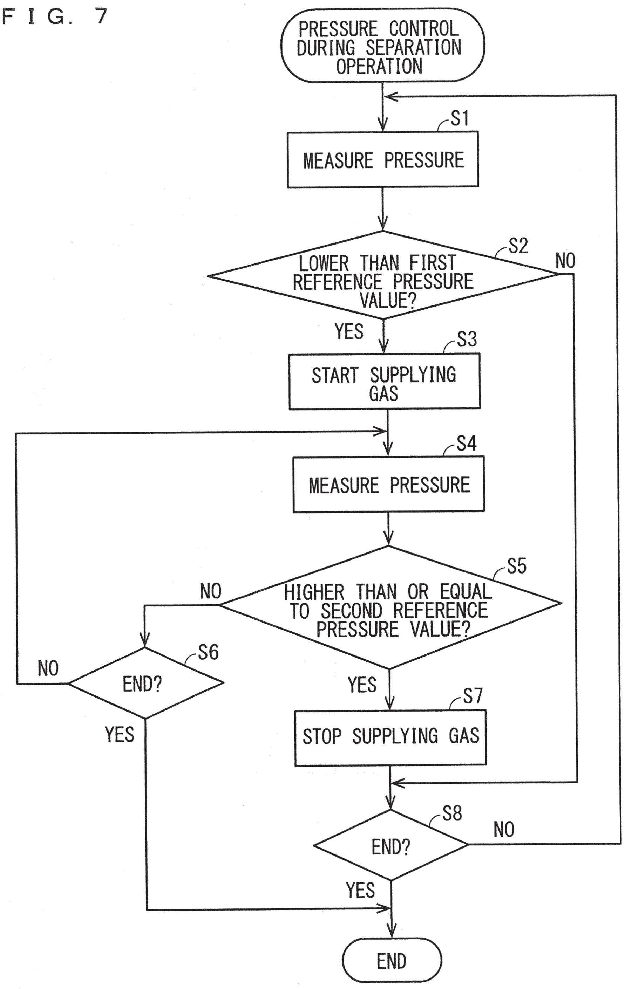

FIG. 7 is a flowchart illustrating example pressure control during the separation operation. For example, the controller 9 starts pressure control upon start of the separation operation. In other words, the controller 9 executes the pressure control in parallel with the separation operation. In the pressure control, first, the pressure sensor Sn3 measures the pressure in the collection tank Tk1 (Step S1: a pressure measurement step). The pressure sensor Sn3 outputs the measured pressure to the controller 9.

Next, the controller 9 determines whether the pressure measured by the pressure sensor Sn3 is lower than the first reference pressure value (Step S2: a pressure determining step). The first reference pressure value is a value larger than a lower limit value of a pressure range in a specification of the collection tank Tk1, and is, for example, set in advance. Data indicating the first reference pressure value is stored in, for example, the storage 94.

When the measured pressure is higher than or equal to the first reference pressure value, the controller 9 determines whether to end the pressure control (Step S8: an end determining step). When determining to end the pressure control, the controller 9 ends the pressure control. The controller 9 may end the pressure control, for example, in response to the end of the separation operation. As a specific example, the controller 9 may end the separation operation and the pressure control when the solvent concentration of the mixed liquid is higher than or equal to the target concentration. When the controller 9 determines not to end the pressure control yet in Step S8, Step S1 is executed again.

When the measured pressure is lower than the first reference pressure value in Step S2, the gas supply part 57 starts supplying gas to the collection tank Tk1 (Step S3: a gas supply starting step). Specifically, the controller 9 opens the gas supply valve 572. Consequently, the gas supply source supplies the gas to the collection tank Tk1 through the first gas supply pipe 571. The pressure in the collection tank Tk1 rises according to a supply amount of the gas. In other words, the gas supply part 57 supplies the gas at a flow rate high enough to increase the pressure in the collection tank Tk1.

Next, the pressure sensor Sn3 measures the pressure in the collection tank Tk1 (Step S4: a pressure measurement step). The pressure sensor Sn3 outputs the measured pressure to the controller 9.

Next, the controller 9 determines whether the pressure measured by the pressure sensor Sn3 is higher than or equal to a second reference pressure value (Step S5: a pressure determining step). The second reference pressure value is set to a value larger than the first reference pressure value. The second reference pressure value is set smaller than an upper limit value of the pressure range in the specification of the collection tank Tk1. Data indicating the second reference pressure value is stored in, for example, the storage 94.

When the measured pressure is lower than the second reference pressure value, the controller 9 determines whether to end the pressure control (Step S6: an end determining step), similarly to Step S8. When the controller 9 determines not to end the pressure control yet, Step S4 is executed again. When determining to end the pressure control, the controller 9 ends the pressure control.

When the measured pressure is higher than or equal to the second reference pressure value in Step S5, the gas supply part 57 stops supplying the gas (Step S7: a gas supply stopping step). Specifically, the controller 9 closes the gas supply valve 572. Consequently, the gas supply source stops supplying the gas to the collection tank Tk1. Next, the controller 9 executes Step S8.

As described above, the gas supply part 57 supplies gas to the collection tank Tk1 when the pressure in the collection tank Tk1 during the separation operation is lower than the first reference pressure value. Thus, the organic solvent regenerator 5 can avoid any further decrease in the pressure in the collection tank Tk1. Consequently, the organic solvent regenerator 5 can raise the pressure in the collection tank Tk1. Thus, an inexpensive tank with low breakdown voltage performance is applicable as the collection tank Tk1. Thus, the manufacturing cost and the size of the collection tank Tk1 can be reduced.

The gas supply valve 572 may be an auto pressure controller. Alternatively, aside from the gas supply valve 572, an auto pressure controller may be disposed in the first gas supply pipe 571. The auto pressure controller regulates the pressure in the collection tank Tk1 in a predetermined pressure range.

### [First reference pressure value]

The first reference pressure value may be set higher than the standard atmosphere. In other words, the first reference pressure value may be of a positive pressure. Consequently, the organic solvent regenerator 5 can maintain the pressure in the collection tank Tk1 at the positive pressure during the separation operation. Thus, the organic solvent regenerator 5 can maintain higher the boiling point of the mixed liquid in the circulation path during the separation operation.

This allows the temperature regulator 65 to regulate the temperature of the mixed liquid to a higher temperature within a temperature range lower than the boiling point. Thus, the organic solvent regenerator 5 can increase the temperature of the mixed liquid and the amount of steam of the mixed liquid more while avoiding a boil of the mixed liquid in the circulation path. Consequently, the organic solvent regenerator 5 can perform separation in the membrane separator 62 with higher efficiency. Specifically, the organic solvent regenerator 5 can improve the increasing speed of the solvent concentration of the mixed liquid.

### [Embodiment 3]

Embodiment 3 is designed to more reliably avoid a decrease in the pressure in the collection tank Tk1.

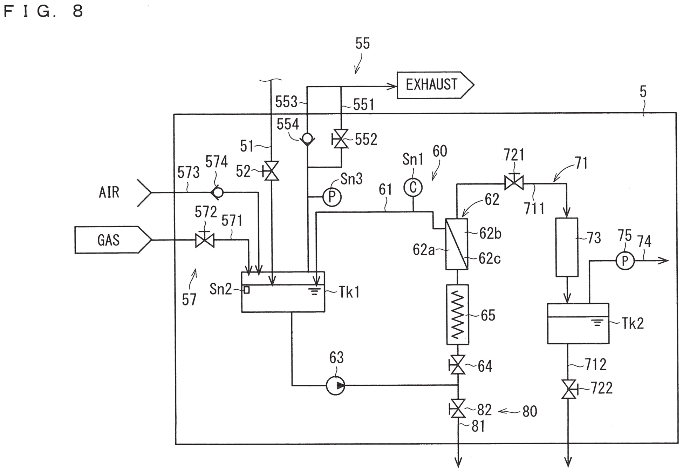

FIG. 8 is a diagram schematically illustrating one example structure of the organic solvent regenerator 5 according to Embodiment 3. The organic solvent regenerator 5 according to Embodiment 3 differs from that according to Embodiment 2 in the structure of the gas supply part 57.

In the example of FIG. 8, the gas supply part 57 further includes a second gas supply pipe 573 and a relief valve 574. A downstream end of the second gas supply pipe 573 is connected to the collection tank Tk1. In the example of FIG. 8, the downstream end of the second gas supply pipe 573 is connected to the ceiling of the collection tank Tk1. A downstream port of the second gas supply pipe 573 is opened above the liquid level of the mixed liquid in the collection tank Tk1. An upstream end of the second gas supply pipe 573 is connected to, for example, open air space (e.g., the interior of a room in a factory). The relief valve 574 is disposed in the second gas supply pipe 573. The relief valve 574 switches between closing and opening of the second gas supply pipe 573. The relief valve 574 is automatically opened when the pressure in the collection tank Tk1 is lower than or equal to a first setting pressure value, and the relief valve 574 is automatically closed when the pressure in the collection tank Tk1 is higher than the first setting pressure value. The first setting pressure value is set to a value smaller than the first reference pressure value and higher than the lower limit value of the pressure range in the specification of the collection tank Tk1. This relief valve 574 is not controlled by the controller 9. Specifically, the relief valve 574 includes a valve seat, and a valve body that receives a first force corresponding to the pressure in the collection tank Tk1. The first force acts on the valve body in a direction toward the valve seat. Furthermore, a second force in a direction away from the valve seat acts on the valve body. When the pressure in the collection tank Tk1 is higher than the first setting pressure value, the first force is greater than the second force, and the valve body is pressed against the valve seat. This closes the relief valve 574. When the pressure in the collection tank Tk1 is lower than or equal to the first setting pressure value, the first force is less than the second force, and the valve body moves in the direction away from the valve seat. This opens the relief valve 574. When the relief valve 574 is opened, the air is supplied to the collection tank Tk1 through the second gas supply pipe 573. Consequently, the pressure in the collection tank Tk1 rises. Thus, any further decrease in the pressure in the collection tank Tk1 can be avoided.

As described above, the second gas supply pipe 573 and the relief valve 574 are provided in Embodiment 3. Even upon occurrence of an abnormality in the gas supply valve 572 or an abnormality in the control function of the controller 9 on the gas supply valve 572, when the pressure in the collection tank tk1 is lower than or equal to the first setting pressure value, the relief valve 574 is automatically opened. Consequently, the organic solvent regenerator 5 can more reliably avoid any further decrease in the pressure in the collection tank Tk1.

The controller 9 may stop the separation operation when the pressure measured by the pressure sensor Sn3 is lower than or equal to the first setting pressure value.

In the aforementioned example, the gas supply part 57 supplies the air to the collection tank Tk1 through the second gas supply pipe 573. Here, the cleanliness of the gas to be supplied to the collection tank Tk1 through the first gas supply pipe 571 may be higher than that of the air to be supplied to the collection tank Tk1 through the second gas supply pipe 573. For example, the number of particles contained in the gas is applicable as an indicator of the cleanliness herein. The fewer the number of particles in the gas is, the higher the cleanliness is.

With this, the controller 9 continues the separation operation while causing the gas supply part 57 to supply cleaner gas to the collection tank Tk1 when the pressure in the collection tank Tk1 is lower than the first reference pressure value and higher than the first setting pressure value. Thus, the organic solvent regenerator 5 can continue the separation operation while mitigating or avoiding contamination of the mixed liquid in the circulation path.

When the pressure in the collection tank Tk1 is lower than or equal to the first setting pressure value, the relief valve 574 is opened, and the controller 9 stops the separation operation. Opening of the relief valve 574 allows the air with low cleanliness to be supplied to the collection tank Tk1. Supply of the air can avoid any further decrease in the pressure in the collection tank Tk1. A filter that captures impurities in the air may be disposed in the second gas supply pipe 573. This allows the cleaner air to be supplied to the collection tank Tk1. Upon occurrence of an abnormality in the filter, there is a danger that the air with low cleanliness flows into the collection tank Tk1. Thus, the controller 9 may stop the separation operation when the pressure in the collection tank Tk1 is lower than or equal to the first setting pressure value. This can avoid an unnecessary separation operation involving an inflow of the air with low cleanliness.

In the example of FIG. 8, the gas discharge part 55 further includes a second exhaust pipe 553 and a relief valve 554. An upstream end of the second exhaust pipe 553 is connected to the collection tank Tk1, and a downstream end of the second exhaust pipe 553 is connected to an exterior exhaust facility. In the example of FIG. 8, a piping portion upstream of the first exhaust pipe 551 serves also as a piping portion upstream of the second exhaust pipe 553, and a piping portion downstream of the first exhaust pipe 551 serves also as a piping portion downstream of the second exhaust pipe 553.

The relief valve 554 is disposed in the second exhaust pipe 553, and switches between closing and opening of the second exhaust pipe 553. In the example of FIG. 8, the relief valve 554 is disposed in a piping portion of the second exhaust pipe 553 which does not serve as the first exhaust pipe 551. The exhaust valve 552 is disposed in a piping portion of the first exhaust pipe 551 which does not serve as the second exhaust pipe 553. The relief valve 554 is automatically opened when the pressure in the collection tank Tk1 is higher than or equal to a second setting pressure value, and the relief valve 554 is automatically closed when the pressure in the collection tank Tk1 is lower than the second setting pressure value. The second setting pressure value is set to a value larger than the second reference pressure value and lower than the upper limit value of the pressure range in the specification of the collection tank Tk1. For example, the relief valve 554 includes a valve seat, and a valve body that receives a third force corresponding to the pressure in the collection tank Tk1. The third force acts on the valve body in a direction away the valve seat. A fourth force also acts on the valve body in a direction toward the valve seat. When the pressure in the collection tank Tk1 is lower than the second setting pressure value, the third force is less than the fourth force, and the relief valve 554 is closed. When the pressure in the collection tank Tk1 is higher than or equal to the second setting pressure value, the third force is greater than the fourth force, and the relief valve 554 is opened. Consequently, the gas in the collection tank Tk1 is discharged to an exterior through the second exhaust pipe 553, so that the pressure in the collection tank Tk1 decreases. Thus, any further pressure rise in the collection tank Tk1 can be more reliably avoided.

### [Embodiment 4]

A structure of the organic solvent regenerator 5 according to Embodiment 4 is identical to any one of the organic solvent regenerators 5 according to Embodiments 1 to 3. In Embodiment 4, the organic solvent regenerator 5 performs a preliminary temperature regulation operation to be described next, immediately before the separation operation. In other words, the preliminary temperature regulation operation is an operation of stopping the decompression pump 75, operating the temperature regulator 65, and causing the circulator 60 to circulate the mixed liquid. Consequently, the organic solvent regenerator 5 can circulate the mixed liquid through the circulation path and heat the mixed liquid, with the separation function of the membrane separator 62 being substantially stopped. In other words, the organic solvent regenerator 5 can heat the mixed liquid before the separation operation.

Before the separation operation, the collection valve 52 is open, and the mixed liquid can flow into the collection tank Tk1. In other words, the organic solvent regenerator 5 performs the preliminary temperature regulation operation with the collection valve 52 being opened. Put it differently, the organic solvent regenerator 5 performs the preliminary temperature regulation operation in a collectable state in which an inflow of the mixed liquid to be discharged from the substrate processing apparatus 1 to the collection tank Tk1 is permitted. To sum up, the organic solvent regenerator 5 performs the preliminary temperature regulation operation during at least a part of the collectable period TC. In other words, the collectable period TC is a period during which the storage amount in the collection tank Tk1 is smaller than the reference collection amount.

As described above, the organic solvent regenerator 5 performs the preliminary temperature regulation operation in parallel with a collection operation of collecting the mixed liquid from the substrate processing apparatus 1 in the collection tank Tk1. Thus, the temperature of the mixed liquid can be raised to a temperature range appropriate for separation, in the separation operation after the collection operation.

Next, closing and opening of the exhaust valve 552 during the preliminary temperature regulation operation will be described. As described in Embodiment 1, when the exhaust valve 552 is open during the inflow period in which at least one of pure water and an organic solvent flows into the collection tank Tk1, the mixed liquid can more easily flow into the collection tank Tk1.

In the preliminary temperature regulation operation, the temperature regulator 65 heats the mixed liquid. Thus, the amount of steam of the organic solvent in the circulation path increases. When the exhaust valve 552 is always open, the steam of the circulating organic solvent is always discharged to an exterior through the first exhaust pipe 551. This increases the discharge amount of the steam of the organic solvent through the first exhaust pipe 551 in the preliminary temperature regulation operation.

The substrate processing apparatus 1 does not always continue to discharge the mixed liquid while processing the substrate W. For example, when the substrate processing apparatus 1 is a single-wafer processing apparatus and supplies a chemical solution, pure water, and an organic solvent in this order to the substrate W, the substrate processing apparatus 1 discharges the chemical solution and does not discharge the mixed liquid during a period in which the substrate processing apparatus 1 supplies the chemical solution. Furthermore, the substrate processing apparatus 1 discharges pure water and does not discharge the organic solvent during a period in which the substrate processing apparatus 1 supplies the pure water. Furthermore, the substrate processing apparatus 1 discharges the organic solvent during a period in which the substrate processing apparatus 1 supplies the organic solvent.

Thus, the controller 9 opens the exhaust valve 552 during at least a part of the inflow period in which the mixed liquid flows into the collection tank Tk1, in a preliminary temperature regulation operation period for performing the preliminary temperature regulation operation. Furthermore, the controller 9 closes the exhaust valve 552 during at least a part of a non-inflow period in which the mixed liquid does not flow into the collection tank Tk1, in the preliminary temperature regulation operation period. Hereinafter, an example of a specific structure and specific operations of the substrate processing apparatus 1, and example specific operations of the organic solvent regenerator 5 will be described in this order.

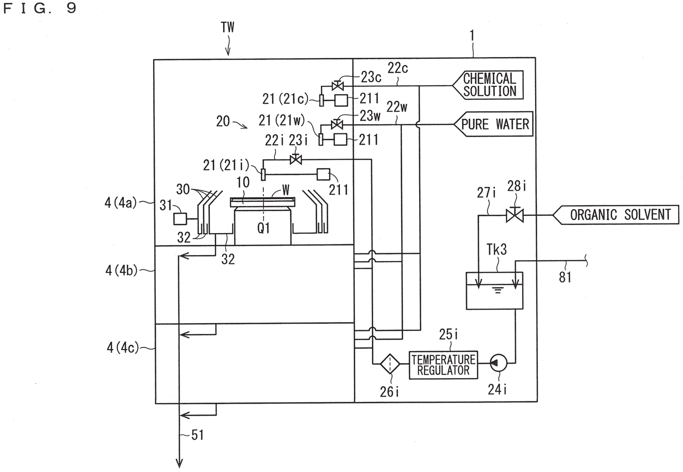

FIG. 9 is a diagram schematically illustrating an example structure of the substrate processing apparatus 1. In the example of FIG. 9, the substrate processing apparatus 1 includes a plurality of processing units 4. Each of the processing units 4 performs a wet process on the substrate W. In the example of FIG. 9, each of the processing units 4 is a single-wafer processing device that processes the substrates W one by one. In the example of FIG. 9, the processing units 4 are stacked in the vertical direction. Here, the processing units 4 form a tower TW. In the example of FIG. 9, a processing unit 4a, a processing unit 4b, and a processing unit 4c are illustrated as the processing units 4. In the example of FIG. 9, the processing unit 4a, the processing unit 4b, and the processing unit 4c form the tower TW. While one tower TW is illustrated in the example of FIG. 9, a plurality of towers TW may be disposed adjacent to each other in the horizontal direction.

The processing unit 4a to the processing unit 4c may have the same structure. In the example of FIG. 9, only the structure of the processing unit 4a is illustrated.

The processing unit 4 includes a substrate holder 10, a dispenser 20, and guards 30. The substrate holder 10 rotates the substrate W about a rotation axis line Q1 while holding the substrate W in a horizontal attitude. The horizontal attitude herein is an attitude in which the thickness direction of the substrate W is along the vertical direction. The rotation axis line Q1 is an axis that passes through the center of the substrate W and is along the vertical direction. This substrate holder 10 can be referred to as a spin chuck. The substrate holder 10 may be a spin chuck of a mechanical chuck type, a vacuum chuck type, or an electrostatic chuck type.

The dispenser 20 sequentially dispenses processing liquids of a plurality of types toward the main surface of the substrate W held by the substrate holder 10. The dispenser 20 dispenses, for example, a chemical solution, pure water, and an organic solvent in this order toward the substrate W. The dispenser 20 includes nozzles 21. In the example of FIG. 9, the dispenser 20 includes a nozzle 21c for chemical solution, a nozzle 21w for pure water, and a nozzle 21i for organic solvent. The nozzle 21c is connected to a chemical solution supply source through a supply pipe 22c. The nozzle 21w is connected to a pure water supply source through a supply pipe 22w. The nozzle 21i is connected to a solvent tank Tk3 through a supply pipe 22i. A supply valve 23c is disposed in the supply pipe 22c. A supply valve 23w is disposed in the supply pipe 22w. A supply valve 23i is disposed in the supply pipe 22i.

In the example of FIG. 9, a liquid feeder 24i, a temperature regulator 25i, and a filter 26i are disposed in the supply pipe 22i. The liquid feeder 24i is a feed pump, and causes the organic solvent to flow from the solvent tank Tk3 toward the nozzle 21i. The temperature regulator 25i regulates the temperature of the organic solvent flowing through the supply pipe 22i. The temperature regulator 25i is, for example, a heater. The filter 26i captures impurities in the organic solvent flowing through the supply pipe 22i.

The solvent tank Tk3 is connected to an organic solvent supply source through a replenish pipe 27i. A replenish valve 28i is disposed in the replenish pipe 27i. Upon opening the replenish valve 28i, the organic solvent is supplied to the solvent tank Tk3 through the replenish pipe 27i.

In the example of FIG. 9, the downstream end of the supply pipe 81 of the organic solvent regenerator 5 is connected to the solvent tank Tk3. In this structure, the concentrate from the organic solvent regenerator 5 is supplied to the solvent tank Tk3 through the supply pipe 81.

In the example of FIG. 9, the processing unit 4 includes nozzle movement drivers 211 that move the respective nozzles 21. Each of the nozzle movement drivers 211 moves the nozzle 21 between a processing position and a waiting position. The processing position is a position at which the nozzle 21 dispenses the processing liquid toward the substrate W, for example, a position facing the center of the substrate W in the vertical direction. The waiting position is a position at which the nozzle 21 does not dispense the processing liquid toward the substrate W, and is, for example, a position outward of the substrate W in the radial direction. The nozzle movement driver 211 includes, for example, a motor.

Once the dispenser 20 dispenses the processing liquid toward the main surface of the rotating substrate W, the processing liquid that sits on the main surface of the substrate W flows outward in the radial direction by centrifugal force of rotation of the substrate W. Then, the processing liquid flies off from the outer edge of the substrate W. When the processing liquid flows through the main surface of the substrate W, a process corresponding to the type of the processing liquid is performed on the substrate W.

The guard 30 is of a tubular shape surrounding the substrate holder 10, and receives the processing liquid that flies off from the outer edge of the substrate W. In the example of FIG. 9, a plurality of the guards 30 are concentrically disposed. A guard lifting driver 31 lifts and lowers the guards 30 between an upper position and a lower position. The upper position is a position at which the upper end of each of the guards 30 is located above the substrate W held by the substrate holder 10. The guard 30 located at the upper position receives the processing liquid that flies off from the outer edge of the substrate W. The lower position is a position at which the upper end of each of the guards 30 is located below the substrate W held by the substrate holder 10. The guards 30 may be used depending on each of the types of the processing liquids. The guard lifting driver 31 includes, for example, a motor.

A cup 32 is disposed below each of the guards 30. The processing liquid received by each of the guards 30 runs down into the cup 32, and is collected by the cup 32. The cups 32 are connected to upstream ends of collection pipes for the respective processing liquids. For example, the cup 32 for organic solvent is connected to an upstream end of the collection pipe 51. In the example of FIG. 9, the cup 32 for organic solvent is the most inner cup 32, and the guard 30 for organic solvent is the most inner guard 30.

The controller 9 controls various structures of the processing units 4 based on recipe information such that processes on the substrate W are performed. The recipe information is information indicating processing details on the substrate W, and includes, for example, information on the dispensing time of a chemical solution, the dispensing time of pure water, and the dispensing time of an organic solvent, the positions of the guards 30 at each timing, and the rotation speed of the substrate W at each timing. For example, the storage 94 stores the recipe information.

For example, the controller 9 first causes the substrate holder 10 to rotate the substrate W about the rotation axis line Q1. The substrate holder 10 may continue to rotate the substrate W until the end of the processes on the substrate W. Then, the dispenser 20 dispenses a chemical solution to the substrate W. Specifically, the controller 9 causes the nozzle movement driver 211 to move the nozzle 21c to the processing position, causes the guard lifting driver 31 to move the guard 30 for chemical solution to the upper position, and opens the supply valve 23c. Consequently, the nozzle 21c dispenses the chemical solution toward the main surface of the rotating substrate W, so that a chemical solution process (e.g., etching) is performed on the main surface of the substrate W. The guard 30 for chemical solution receives the chemical solution that flies off from the outer edge of the substrate W in the chemical solution process. Then, the controller 9 ends the chemical solution process in response to elapsing of the dispensing time of the chemical solution.

Next, the dispenser 20 dispenses pure water. Specifically, the controller 9 causes the nozzle movement driver 211 to move the nozzle 21w to the processing position, causes the guard lifting driver 31 to move the guard 30 for pure water to the upper position, and opens the supply valve 23w. Consequently, the nozzle 21w dispenses pure water, so that the pure water forces the chemical solution out of the main surface of the substrate W (a rinsing process). Thus, the processing liquid on the main surface of the substrate W is replaced from the chemical solution with a rinse liquid. The guard 30 for pure water can receive the pure water that flies off from the outer edge of the substrate W. Then, the controller 9 ends the rinsing process in response to elapsing of the dispensing time of pure water.

Next, the dispenser 20 dispenses the organic solvent. Specifically, the controller 9 causes the nozzle movement driver 211 to move the nozzle 21i to the processing position, causes the guard lifting driver 31 to move the guard 30 for organic solvent to the upper position, and opens the supply valve 23i. Consequently, the nozzle 21i dispenses the organic solvent, so that the organic solvent forces the pure water out of the main surface of the substrate W (an organic solvent process). Thus, the processing liquid on the main surface of the substrate W is replaced from the rinse liquid with the organic solvent. The guard 30 for organic solvent receives the pure water and the organic solvent which fly off from the outer edge of the substrate W. The collection tank Tk1 collects the pure water and the organic solvent through the cups 32 and the collection pipe 51. The controller 9 ends the organic solvent process in response to elapsing of the dispensing time of the organic solvent.

Next, the processing unit 4 dries the substrate W. Specifically, the controller 9 improves the rotation speed of the substrate holder 10 (i.e., spin drying). During this drying period, the guard 30 for organic solvent is located at the upper position, and receives the organic solvent that flies off from the outer edge of the substrate W. Thus, the collection tank Tk1 collects the organic solvent through the collection pipe 51 during the drying period.

In the aforementioned example, during a solvent period in which the nozzle 21i dispenses the organic solvent, initially, the organic solvent forces the pure water out of the main surface of the substrate W, and the guard 30 for organic solvent receives the pure water and the organic solvent which fly off from the outer edge of the substrate W. Once the organic solvent forces most of the entirety of the pure water out of the main surface of the substrate W, the guard 30 receives virtually only the organic solvent in the subsequent times. During this solvent period, the pure water and the organic solvent which have been received by the guards 30 are discharged to the collection pipe 51 through the cups 32, and collected in the collection tank Tk1 through the collection pipe 51. During the drying period, the organic solvent received by the guard 30 for organic solvent is discharged to the collection pipe 51 through the cup 32, and collected in the collection tank Tk1 through the collection pipe 51.

As described above, the processing unit 4 discharges the mixed liquid to the collection pipe 51 during the solvent period and the drying period in a processing period during which the processing unit 4 processes the substrate W. The processing unit 4 does not discharge the mixed liquid to the collection pipe 51 during a period except the solvent period and the drying period.

In the example of FIG. 9, the processing units 4 forming the one tower TW are connected to the common collection pipe 51. In other words, the cup 32 for organic solvent of the processing unit 4a, the cup 32 for organic solvent of the processing unit 4b, and the cup 32 for organic solvent of the processing unit 4c are connected to the common collection pipe 51. In this structure, when the pure water and the organic solvent from at least one of the processing unit 4a, the processing unit 4b, and the processing unit 4c are discharged to the collection pipe 51, the pure water and the organic solvent flow into the collection tank Tk1 through the collection pipe 51. When none of the processing unit 4a, the processing unit 4b, and the processing unit 4c discharges the pure water and the organic solvent to the collection pipe 51, neither the pure water nor the organic solvent flows into the collection tank Tk1.

Thus, the controller 9 according to Embodiment 4 opens the exhaust valve 552 during at least a part of the inflow period in which at least one of the pure water and the organic solvent from the substrate processing apparatus 1 flows into the collection tank Tk1. Furthermore, the controller 9 closes the exhaust valve 552 during at least a part of the non-inflow period in which neither the pure water nor the organic solvent flows into the collection tank Tk1.

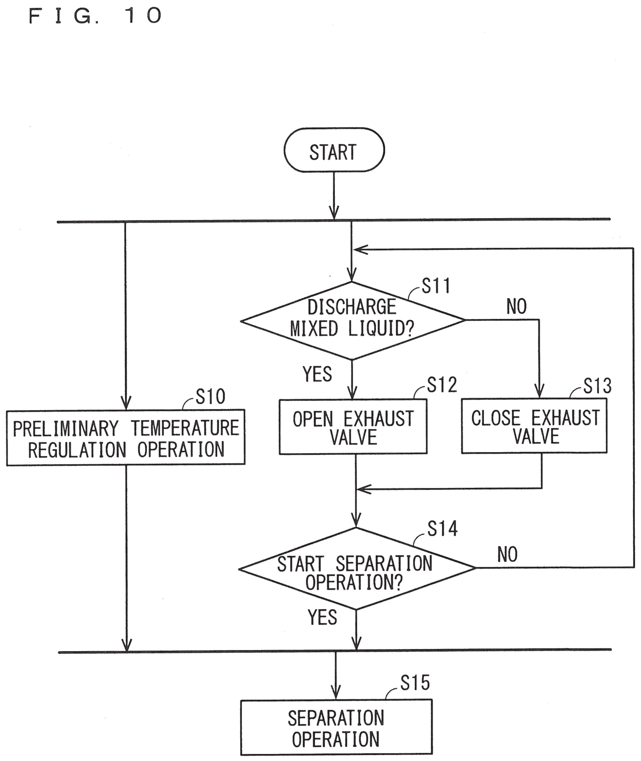

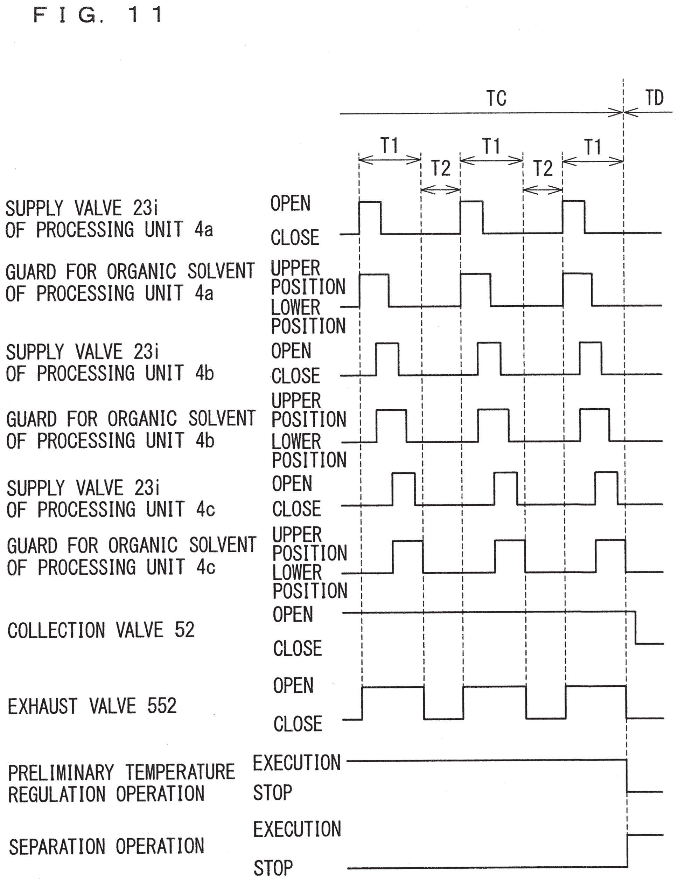

FIG. 10 is a flowchart illustrating example operations of the organic solvent regenerator 5 according to Embodiment 4. FIG. 11 is a timing chart illustrating example operations of the substrate processing system 100 according to Embodiment 4. Initially, the storage amount of the mixed liquid in the collection tank Tk1 is smaller than the reference collection amount yet, and the controller 9 opens the collection valve 52. In other words, the organic solvent regenerator 5 performs the collection operation in the collection tank Tk1.

In the example of FIG. 10, the organic solvent regenerator 5 performs the preliminary temperature regulation operation (Step S10) before the separation operation (Step S15). Specifically, the controller 9 opens the circulation valve 64 and operates the liquid feeder 63 and the temperature regulator 65, while closing the separation valve 721 and stopping the decompression pump 75. Consequently, the mixed liquid is heated by the temperature regulator 65 and circulated through the circulation path, while the separation function of the membrane separator 62 is not substantially implemented. This preliminary temperature regulation operation is performed in parallel with the collection operation.

In the example of FIG. 10, Steps S11 to S14 are executed in parallel with the preliminary temperature regulation operation. In Step S11, the controller 9 determines whether at least one of the pure water and the organic solvent is discharged to the collection pipe 51. In other words, the controller 9 determines whether at least one of the pure water and the organic solvent flows into the collection tank Tk1. For example, when the guard 30 for organic solvent in any one of the processing units 4 is located at the upper position, the controller 9 may determine that at least one of the pure water and the organic solvent is discharged to the collection pipe 51. For example, when the guard 30 for organic solvent in each of the processing units 4 is located at the lower position, the controller 9 may determine that the substrate processing apparatus 1 discharges none of the pure water and the organic solvent to the collection pipe 51.

When at least one of the pure water and the organic solvent is discharged to the collection pipe 51, the controller 9 opens the exhaust valve 552 (Step S12). For example, when the guard 30 for organic solvent in at least one of the processing unit 4a, the processing unit 4b, and the processing unit 4c is located at the upper position as illustrated in FIG. 11, that is, in inflow periods T1, the controller 9 opens the exhaust valve 552. Consequently, at least one of the pure water and the organic solvent discharged from at least one of the processing unit 4a, the processing unit 4b, and the processing unit 4c can easily flow into the collection tank Tk1 through the collection pipe 51, similarly to Embodiment 1. The controller 9 may open the exhaust valve 552 during only a part of the inflow period T1, while the controller 9 opens the exhaust valve 552 during the entirety of the inflow period T1 in the example of FIG. 11. A period during which the exhaust valve 552 is open may be, for example, half the inflow period T1 or longer, 80% of the inflow period T1 or more, 90% of the inflow period T1 or more, or the entirety of the inflow period T1.

When none of the pure water and the organic solvent is discharged to the collection pipe 51 in Step S11, the controller 9 closes the exhaust valve 552 (Step S13). For example, when the guard 30 for organic solvent in each of the processing unit 4a, the processing unit 4b, and the processing unit 4c is located at the lower position as illustrated in FIG. 11, that is, in non-inflow periods T2, the controller 9 closes the exhaust valve 552. When the exhaust valve 552 is closed, the steam of the circulating organic solvent is not discharged to an exterior through the first exhaust pipe 551. This can reduce the amount of steam of the organic solvent to be discharge through the first exhaust pipe 551 in the entirety of the preliminary temperature regulation operation. The controller 9 may close the exhaust valve 552 during only a part of the non-inflow period T2, while the controller 9 closes the exhaust valve 552 during the entirety of the non-inflow period T2 in the example of FIG. 11. A period during which the exhaust valve 552 is closed may be, for example, half the non-inflow period T2 or longer, 80% of the non-inflow period T2 or more, 90% of the non-inflow period T2 or more, or the entirety of the non-inflow period T2.

In Step S14 after Step S12 or Step S13, the controller 9 determines whether to start the separation operation. For example, when the storage amount of the mixed liquid in the collection tank Tk1 is larger than or equal to a predetermined reference collection amount, the controller 9 determines to start the separation operation. When the storage amount is smaller than the reference collection amount yet, the controller 9 determines not to start the separation operation yet. When determining not to start the separation operation yet, the controller 9 executes Step S11 again.

When determining to start the separation operation in Step S14, the controller 9 starts the separation operation in Step S15. For example, the controller 9 first closes the collection valve 52 and the exhaust valve 552. Then, the controller 9 opens the separation valve 721, and causes the circulator 60 to circulate the mixed liquid while operating the decompression pump 75.

As described above, the organic solvent regenerator 5 performs the preliminary temperature regulation operation in parallel with the collection operation (Step S10) before the separation operation in Embodiment 4. Thus, the temperature of the mixed liquid can reach the temperature range appropriate for the separation operation at an earlier timing after the separation operation is started. Alternatively, the temperature of the mixed liquid can reach the temperature range appropriate for the separation operation when the separation operation is started. Thus, the organic solvent regenerator 5 can raise the solvent concentration of the mixed liquid to or above the target concentration at an earlier timing.

The controller 9 opens the exhaust valve 552 during at least a part of the inflow periods T1 in the preliminary temperature regulation operation period. Thus, at least one of the pure water and the organic solvent from the substrate processing apparatus 1 easily flows into the collection tank Tk1. Furthermore, the controller 9 closes the exhaust valve 552 during at least a part of the non-inflow periods T2 in the preliminary temperature regulation operation period. This can reduce the amount of steam of the organic solvent to be discharged to an exterior through the first exhaust pipe 551 during the preliminary temperature regulation operation period.

The organic solvent regenerator 5 need not perform the preliminary temperature regulation operation during the entirety of a period in which the storage amount of the mixed liquid in the collection tank Tk1 is smaller than the reference collection amount. For example, the organic solvent regenerator 5 may start the preliminary temperature regulation operation when the storage amount of the mixed liquid in the collection tank Tk1 exceeds a reference storage amount smaller than the reference collection amount.

While the organic solvent regenerator 5, and the substrate processing system 100, and the method of regenerating an organic solvent are described in detail above, the description is in all aspects illustrative and does not restrict this disclosure. The aforementioned various modifications are applicable in combination unless any contradiction occurs. Therefore, numerous modifications and variations that have not yet been exemplified are devised without departing from the scope of the present disclosure.

The present disclosure includes the following aspects.

A first aspect is an organic solvent regenerator including: a collection tank into which an organic solvent and water flow through a collection pipe and which stores a mixed liquid of the organic solvent and the water, the organic solvent and the water being discharged from a substrate processing apparatus that processes a substrate; an exhaust pipe connected to the collection tank; an exhaust valve disposed in the exhaust pipe; a circulator including a circulation pipe connected to the collection tank, and a membrane separator disposed in the circulation pipe, the membrane separator separating the water from the mixed liquid; a separation pipe which is connected to the membrane separator and through which the water separated by the membrane separator flows; and a controller that opens the exhaust valve during at least a part of an inflow period in which at least one of the water and the organic solvent flows into the collection tank through the collection pipe and that closes the exhaust valve during at least a part of a separation operation period in which a separation operation of causing the circulator to circulate the mixed liquid and separating the water from the mixed liquid is performed.

A second aspect is the organic solvent regenerator according to the first aspect which includes: a pressure sensor that measures a pressure in the collection tank; a first gas supply pipe which is connected to the collection tank and through which gas flows toward the collection tank; and a gas supply valve disposed in the first gas supply pipe, wherein the controller opens the gas supply valve when the pressure in the collection tank is lower than a first reference pressure value in the separation operation.

A third aspect is the organic solvent regenerator according to the second aspect, wherein the circulator further includes a temperature regulator that heats the mixed liquid flowing through the circulation pipe, the controller causes the temperature regulator to heat the mixed liquid in the separation operation, and the first reference pressure value is higher than or equal to a standard atmosphere.

A fourth aspect is the organic solvent regenerator according to the second or third aspect which includes: a second gas supply pipe which is connected to the collection tank and through which air flows toward the collection tank; and a relief valve that is disposed in the second gas supply pipe and that is opened when the pressure in the collection tank falls below a setting pressure value smaller than the first reference pressure value.

A fifth aspect is the organic solvent regenerator according to any one of the first to fourth aspects, wherein the membrane separator includes a first path inserted into the circulation pipe, a second path connected to the separation pipe, and a separation membrane that partitions the first path and the second path, the organic solvent regenerator comprises a decompression pump that reduces a pressure in the second path through the separation pipe, the circulator further includes a temperature regulator that heats the mixed liquid flowing through the circulation pipe, the controller starts the separation operation when a storage amount of the mixed liquid in the collection tank is larger than or equal to a reference collection amount, the controller performs a preliminary temperature regulation operation of stopping the decompression pump, operating the temperature regulator, and causing the circulator to circulate the mixed liquid during at least a part of a collectable period in which the storage amount of the mixed liquid in the collection tank is smaller than the reference collection amount, and the controller closes the exhaust valve during at least a part of a non-inflow period in which none of the water and the organic solvent flows into the collection tank in a preliminary temperature regulation operation period during which the preliminary temperature regulation operation is performed, and opens the exhaust valve during at least a part of an inflow period in which at least one of the water and the organic solvent flows into the collection tank in the preliminary temperature regulation operation period.

A sixth aspect is the substrate processing system including: the organic solvent regenerator according to any one of the first to fifth aspects; and the substrate processing apparatus.

A seventh aspect is a method of regenerating an organic solvent, and the method includes: opening an exhaust valve disposed in an exhaust pipe connected to a collection tank during at least a part of an inflow period in which at least one of an organic solvent and water flows into the collection tank through a collection pipe, the organic solvent and the water being discharged from a substrate processing apparatus that processes a substrate; and closing the exhaust valve during at least a part of a separation operation period in which a separation operation of causing a circulator to circulate a mixed liquid of the organic solvent and the water and separating the water from the mixed liquid is performed, the circulator including a circulation pipe connected to the collection tank storing the mixed liquid, and a membrane separator disposed in the circulation pipe and separating the water from the mixed liquid.

According to the first, sixth, and seventh aspects, the exhaust valve is opened during at least a part of the inflow period. When the exhaust valve is opened, an inflow of the mixed liquid into the collection tank causes the gas in the collection tank to be discharged through the exhaust pipe. Thus, the mixed liquid easily flows into the collection tank. The exhaust valve is closed during at least a part of the separation operation period. This can reduce the amount of steam of the circulating organic solvent to be discharged to an exterior through an exhaust pipe. Thus, a waste amount of the organic solvent can be reduced.

According to the second aspect, the separation operation causes water in the mixed liquid to flow from the circulation path into the separation pipe. Thus, the pressure in the collection tank decreases according to an inflow amount of water in the separation pipe. Thus, the controller opens the gas supply valve when the pressure in the collection tank is lower than the first reference pressure value. Consequently, any further decrease in the pressure in the collection tank can be avoided.

According to the third aspect, since the boiling point of the circulating mixed liquid can be set high, the temperature of the mixed liquid can be set higher while avoiding a boil of the mixed liquid during the separation operation. Thus, the amount of steam of the circulating mixed liquid can be increased. Consequently, the membrane separator can separate a separation fluid (water vapors) from the mixed liquid more efficiently.

According to the fourth aspect, a decrease in the pressure in the collection tank in a region lower than a setting pressure value can be more reliably avoided.

According to the fifth aspect, the exhaust valve is closed during at least a part of the non-inflow period. This can reduce the amount of steam of the organic solvent to be discharged to an exterior through an exhaust pipe in the preliminary temperature regulation operation. In contrast, the exhaust valve is opened during at least a part of the inflow period. Thus, the mixed liquid easily flows into the collection tank.

## Claims

1. An organic solvent regenerator, comprising:
a collection tank (Tk1) into which an organic solvent and water flow through a collection pipe (51) and which stores a mixed liquid of the organic solvent and the water, the organic solvent and the water being discharged from a substrate processing apparatus (1) that processes a substrate (W);
an exhaust pipe (551) connected to the collection tank (Tk1);
an exhaust valve (552) disposed in the exhaust pipe (551);
a circulator (60) including a circulation pipe (61) connected to the collection tank (Tk1), and a membrane separator (62) disposed in the circulation pipe (61), the membrane separator (62) separating the water from the mixed liquid;
a separation pipe (71) which is connected to the membrane separator (62) and through which the water separated by the membrane separator (62) flows; and
a controller (9) that opens the exhaust valve (552) during at least a part of an inflow period in which at least one of the water and the organic solvent flows into the collection tank (Tk1) through the collection pipe (51) and that closes the exhaust valve (552) during at least a part of a separation operation period in which a separation operation of causing the circulator (60) to circulate the mixed liquid and separating the water from the mixed liquid is performed.

2. The organic solvent regenerator according to claim 1, comprising:
a pressure sensor (Sn3) that measures a pressure in the collection tank (Tk1);
a first gas supply pipe (571) which is connected to the collection tank (Tk1) and through which gas flows toward the collection tank (Tk1); and
a gas supply valve (572) disposed in the first gas supply pipe (571),
wherein the controller (9) opens the gas supply valve (572) when the pressure in the collection tank (Tk1) is lower than a first reference pressure value in the separation operation.

3. The organic solvent regenerator according to claim 2,
wherein the circulator (60) further includes a temperature regulator (65) that heats the mixed liquid flowing through the circulation pipe (61),
the controller (9) causes the temperature regulator (65) to heat the mixed liquid in the separation operation, and
the first reference pressure value is higher than or equal to a standard atmosphere.

4. The organic solvent regenerator according to claim 2 or 3, comprising:
a second gas supply pipe (573) which is connected to the collection tank (Tk1) and through which air flows toward the collection tank (Tk1); and
a relief valve (574) that is disposed in the second gas supply pipe (573) and that is opened when the pressure in the collection tank (Tk1) falls below a setting pressure value smaller than the first reference pressure value.

5. The organic solvent regenerator according to any one of claims 1 to 4,
wherein the membrane separator (62) includes a first path (62a) inserted into the circulation pipe (61), a second path (62b) connected to the separation pipe (71), and a separation membrane (62c) that partitions the first path (62a) and the second path (62b),
the organic solvent regenerator comprises a decompression pump (75) that reduces a pressure in the second path (62b) through the separation pipe (71),
the circulator (60) further includes a temperature regulator (65) that heats the mixed liquid flowing through the circulation pipe (61),
the controller (9) starts the separation operation when a storage amount of the mixed liquid in the collection tank (Tk1) is larger than or equal to a reference collection amount,
the controller (9) performs a preliminary temperature regulation operation of stopping the decompression pump (75), operating the temperature regulator (65), and causing the circulator (60) to circulate the mixed liquid during at least a part of a collectable period in which the storage amount of the mixed liquid in the collection tank (Tk1) is smaller than the reference collection amount, and
the controller (9) closes the exhaust valve (552) during at least a part of a non-inflow period in which none of the water and the organic solvent flows into the collection tank (Tk1) in a preliminary temperature regulation operation period during which the preliminary temperature regulation operation is performed, and opens the exhaust valve (552) during at least a part of an inflow period in which at least one of the water and the organic solvent flows into the collection tank (Tk1) in the preliminary temperature regulation operation period.

6. A substrate processing system, comprising:
the organic solvent regenerator (5) according to any one of claims 1 to 5; and
the substrate processing apparatus (1).

7. A method of regenerating an organic solvent, the method comprising:
opening an exhaust valve (552) disposed in an exhaust pipe (551) connected to a collection tank (Tk1) during at least a part of an inflow period in which at least one of an organic solvent and water flows into the collection tank (Tk1) through a collection pipe (51), the organic solvent and the water being discharged from a substrate processing apparatus (1) that processes a substrate (W); and
closing the exhaust valve (552) during at least a part of a separation operation period in which a separation operation of causing a circulator (60) to circulate a mixed liquid of the organic solvent and the water and separating the water from the mixed liquid is performed, the circulator (60) including a circulation pipe (61) connected to the collection tank (Tk1) storing the mixed liquid, and a membrane separator (62) disposed in the circulation pipe (61) and separating the water from the mixed liquid.
